# EUROPÄISCHE PATENTSCHRIFT

(11) **EP 3 875 950 B1**
(45) Veröffentlichungstag und Bekanntmachung des Hinweises auf die Patenterteilung: **30.09.2026**
(21) Anmeldenummer: 20161149.8
(22) Anmeldetag: 05.03.2020
(51) Int. Cl.: G01N 27/416, G01N 33/18

(54) **BESTIMMUNG VON CHLORAT MIT EINER ELEKTRODE SOWIE VERFAHREN UND VORRICHTUNG ZUR KALIBRIERUNG DER ELEKTRODE**
DETERMINATION OF CHLORATE WITH AN ELECTRODE AND METHOD AND APPARATUS FOR CALIBRATING THE ELECTRODE
DÉTERMINATION DE CHLORATE DOTÉ D'UNE ÉLECTRODE AINSI QUE PROCÉDÉ ET DISPOSITIF D'ÉTALONNAGE DE L'ÉLECTRODE

(43) Veröffentlichungstag der Anmeldung: 08.09.2021
(73) Patentinhaber: Carela GmbH, 79618 Rheinfelden (DE)
(72) Erfinder: KRUMREY, Bernd, 79591 Eimeldingen (DE); KRUMREY, Julian, 79540 Lörrach (DE)
(74) Vertreter: Friese Goeden Patentanwälte PartGmbB

(56) Entgegenhaltungen:
- EP-A1- 1 484 606
- EP-A1- 1 788 382
- EP-A1- 2 284 531
- EP-A1- 2 557 419
- WO-A1-2009/057034
- WO-A1-2018/230660
- JP-A- 2004 085 450
- JP-A- 2005 291 994
- US-A- 5 948 236
- US-A1- 2011 198 239
- HOSSEINI SEYED GHORBAN ET AL: "Electrochemical detection of chlorate on a novel nano-Au/TiO2NT electrode", MATERIALS RESEARCH BULLETIN, vol. 93, 15 May 2017 (2017-05-15), pages 290 - 295, XP085063251, ISSN: 0025-5408, DOI: 10.1016/J.MATERRESBULL.2017.05.034

## Beschreibung

Die Erfindung betrifft ein Verfahren und eine Vorrichtung zur Kalibrierung eines 2- oder 3-Elektrodensystems.

Chlorat ist ein toxikologisch nicht unbedenkliches Ion. Wenn Chlorat in Lebensmitteln oder Trinkwasser vorkommt, kann es zu Risiken für die öffentliche Gesundheit kommen. Vor allem mit Chlorat belastetes Trinkwasser kann bei allen Altersgruppen zur chronischen Aufnahme von Chlorat beitragen (Breitling-Utzmann, C.: Chlorat in Trinkwasser - Ein Update mit neuen Höchstwerten, https://www.ua-bw.de/pub/beitrag.asp?subid=1&Thema_ID=2&ID=2714&lang=DE&Pdf =No; abgerufen am 02.03.2020).

Chlorat kann bei der Desinfektion von Trinkwasser entstehen, also bei Prozessen, die eigentlich dazu dienen, Wasser für den menschlichen Gebrauch aufzubereiten. Chlorat wird deshalb als Desinfektionsnebenprodukt bezeichnet. Der Nachweis erfolgte bisher über die sogenannte Ionenchromatographie, die sich als wichtigste Analysetechnik etabliert hat (Makart S. und Jensen D., Tirnkwasseranalytik - Desinfektionsnebenprodukte unerwünscht https://www.git-labor.de/applikationen/analytik/trinkwasseranalytikdesinfektionsnebenprodukte-unerwuenscht, abgerufen am 02.03.2020).

In der EU-Trinkwasserverordnung vom Februar 2020 wurde deshalb ein Grenzwert von 0,25 mg/l für Chlorat festgelegt. Ein parametrischer Wert von 0,7 mg/l ist anzuwenden, wenn eine Desinfektionsmethode unter Einsatz von Chlorat zur Desinfektion von Wasser für den menschlichen Gebrauch verwendet wird. Die Mitgliedstaaten streben nach Möglichkeit einen niedrigeren Wert an, ohne die Desinfektion zu beeinträchtigen.

Bei der Ionenchromatographie erfolgt die Trennung an einer Trennsäule, die ein organisches Polymerharz umfasst. Die Detektion kann mittels Leitfähigkeitsdetektion erfolgen. In einem Chromatogramm wird das Detektionssignal als Funktion der Zeit dargestellt. Die Chromatographie kann 35 Minuten und auch länger dauern (Hübenbecker, Untersuchung zur Entstehung von Desinfektionsnebenprodukten bei der Aufbereitung von Trinkwasser an Bord schwimmender Marineeinheiten unter Anwendungsbedingungen, Dissertation, Bonn, 2010, Seite 32 ff.). Nachteilig an der Ionenchromatographie ist der relativ hohe apparative Aufwand und die lange Dauer bis Daten erhalten werden. Außerdem ist dieses Verfahren störanfällig.

Verfahren und Systeme zur Wasseranalyse bzw. zur Bestimmmung von chlorhaltigen Desinfektionsnebenprodukten in Wasser wie Chlorat, Chlorit, Chlor sind beispielsweise beschrieben in: EP 1 788 382 A1; WO 2018/230660 A1; EP 1 484 606 A1; EP 2 557 419 A1; Hosseini Seyed Ghorban et al, Electrochemical detection of chlorate on a novel nano-Au/TiO2NT electrode, Materials Research Bulletin, Bd. 93, 2017, S. 290-295; US 5 948 236 A; US 2011/198239 A1; WO 2009/057034 A1; EP 2 284 531 A1; JP 2004 085450 A; JP 2005 291994 A. Ausgehend vom Stand der Technik liegt der Erfindung somit die Aufgabe zugrunde, die Detektion von Chlorat in einfacher und schneller Weise zu ermöglichen, wobei der apparative Aufwand gering ist, die Daten nach kurzer Zeit erhalten werden und der Nachweis zuverlässig möglich ist.

Die Aufgabe wird erfindungsgemäß durch ein Kalibrierverfahren nach Anspruch 1 und eine Kalibriervorrichtung nach Anspruch 2 gelöst. 2- oder 3-Elektrodensysteme sind an sich bekannt und auch kommerziell erhältlich. Es handelt sich dabei um solche 2- oder 3-Elektrodensysteme, wie sie bisher zum Nachweis von Chlor, Chlorit, Chlordioxid, Ozon und Peressigsäure verwendet wurden. Diese können falls erforderlich angepasst werden, um Chlorat zu bestimmen, beispielsweise durch Wahl eines Elektrolyten und/oder einer Membran. Dies kann in einfacher Weise vom Fachmann in Routineexperimenten ermittelt werden.

Unter dem Begriff "Bestimmung von Chlorat" im Sinne der vorliegenden Erfindung wird insbesondere die Bestimmung der Konzentration von Chlorat in wässrigen Medien verstanden.

Das erfindungsgemäße Verfahren zum Kalibrieren eines 2- oder 3-Elektrodensystems, das zur Bestimmung der Konzentration von Chlorat in Wasser eingesetzt wird, weist die in Anspruch 1 angegebenen Merkmale auf.

Neben dem 2- oder 3-Elektrodensystem, mit dem Chlorat bestimmt wird, kann noch eine oder mehrere weiter Messsonden vorliegen, die insbesondere zur Bestimmung von Chlor, Chlorit, Chlordioxid, Ozon und/oder Peressigsäure eingesetzt werden können.

Mit diesem Verfahren ist es möglich, auch bei kleinen zu desinfizierenden Wassermengen, bei schwankender Konzentration von Chlorat/Desinfektionswirkstoffen und bei unregelmäßigen Wasserabnahmen auch zu Beginn einer Desinfektionsmaßnahme die amperometrische Messung der Konzentration von Chlorat/Desinfektionswirkstoffen zuverlässig zu kalibrieren.

Chlorat wird hier neben den Desinfektionswirkstoffen angegeben, da es sich dabei um ein Desinfektionsnebenprodukt handelt.

Die Kalibrierung ist in der Messtechnik ein Messprozess zur zuverlässig reproduzierbaren Feststellung und Dokumentation der Abweichung eines Messgerätes oder einer Maßverkörperung gegenüber einem anderen Gerät oder einer anderen Maßverkörperung, die in diesem Fall als Normal bezeichnet wird. Beim erfindungsgemäßen Verfahren ist das Messgerät, das kalibriert werden soll, das 2- oder 3-Elektrodensystem für die Bestimmung der Konzentration von Chlorat in Wasser. Das Normal ist z.B. die Bestimmung der Konzentration von Desinfektionswirkstoffen in Wasser mit einem anderen, diskontinuierlichen Verfahren, beispielsweise mit dem DPD-Verfahren, bei welchem das Reagenz N,N-Diethyl-p-phenylendiamin eingesetzt wird. Es bildet beispielsweise mit freiem Chlor (Desinfektionswirkstoff) eine rötliche Färbung, deren Intensität proportional zur Konzentration des freien Chlors ist und photometrisch gemessen werden kann. Das Photometer wird mit externen Farbstandards geprüft. Auf diese Weise kann mit einer chemischen Reaktion die Konzentration von Chlorat in Wasser bestimmt werden. Diese Bestimmung und der damit erhaltene Wert wird als Normal für die Kalibrierung eingesetzt. Mit dem in Schritt (c) bestimmten Normal kann der Messwert des 2- oder 3-Elektrodensystems mit der Konzentration von Chlorat korreliert werden. Der Fachmann wird das Prinzip leicht auf weitere Nachweismethoden oder andere Reagenzien für andere Desinfektionswirkstoffe und Desinfektionsnebenprodukten, wie Chlorat, übertragen können.

In der Definition des VIM von JCGM 2008 gehört zur Kalibrierung üblicherweise ein zweiter Schritt, nämlich die Berücksichtigung der ermittelten Abweichung bei der anschließenden Benutzung des Messgerätes zur Korrektur der abgelesenen Werte. Dieser zweite Schritt ist beim erfindungsgemäßen Verfahren optional. Die Kalibrierung kann ohne Eingriff erfolgen, der das Messgerät verändert.

Bei dem in Schritt (a) genannten zu untersuchenden Wasser kann es sich um das Wasser handeln, in welchem die Konzentration von Chlorat und ggf. Desinfektionswirkstoffen bestimmt werden soll. Im Falle einer Trinkwasserversorgung kann dieses Wasser beispielsweise unmittelbar einer Wasserleitung bzw. dem Ablauf eines Hochbehälters entnommen werden. Im ersten Betriebszustand, welcher den Normalbetrieb darstellt, kann die Konzentration von Chlorat und ggf. Desinfektionswirkstoffen mit dem 2- oder 3-Elektrodensystem bestimmt werden und das Wasser der Hauptleitung wieder zugeführt werden. Bei kleineren Trinkwasserversorgungen kann auch der gesamte Wasserstrom über das 2- oder 3-Elektrodensystem geführt werden. Im zweiten Betriebszustand, welcher den Kalibrierbetrieb darstellt, wird das entnommene Wasser in einen Kreislauf eingespeist, in dem sich das zu kalibrierende 2- oder 3-Elektrodensystem befindet und dort bis zum Abschluss der Kalibrierung zirkuliert. Somit ist der Wasserkreislauf für Schritt (a) des erfindungsgemäßen Verfahrens zumindest teilweise verschieden von der Wasserleitung, dessen Wasser untersucht werden soll. Durch das Aufrechterhalten einer Strömung des zu untersuchenden Wassers im Kreislauf wird eine genaue Kalibrierung möglich, da die Abweichung der Messwerte einer ruhenden Wasserprobe von denen eines umströmten 2- oder 3-Elektrodensystems vermieden wird. Da andrerseits das Wasser im Kreislauf zirkuliert wird, wird sich dessen Zusammensetzung und insbesondere die Konzentration von Chlorat und ggf. Desinfektionswirkstoffen bis zum Abschluss der als Normal verwendeten Bestimmung der Konzentration von Desinfektionswirkstoffen mit einem anderen, diskontinuierlichen Verfahren nicht ändern. Zur Zirkulation kann eine elektrisch angetriebene Pumpe verwendet werden.

In einer Ausführungsform kann in Schritt (b) der Messwert bestimmt werden, wenn er einen etwa konstanten Wert erreicht hat. Ein solcher konstanter Wert kann ein Wert sein, bei dem der gemessene Wert nur noch um etwa ± 10 % oder etwa ± 5 % oder etwa ± 3 % oder etwa ± 2 % oder etwa ± 1 % schwankt. Auf diese Weise kann sichergestellt werden, dass durch Konzentrationsschwankungen verursachte Fehler vermieden werden.

In einer Ausführungsform kann in Schritt (c) die Konzentration von Chlorat und (ggf. Desinfektionswirkstoffen mit der N,N-Diethyl-p-phenylendiamin(DPD)-Methode) bestimmt werden, wie es vorstehend erläutert wurde. Die Konzentration des Desinfektionswirkstoffs in der Probe kann somit verlässlich bestimmt werden. Dieser in Schritt (c) bestimmte Wert dient als Normal, um das 2- oder 3-Elektrodensystem auf der Basis des in Schritt (b) gewonnenen Messwertes zu kalibrieren.

In einer Ausführungsform kann weiterhin vom abgezweigten Wasser im Kreislauf die Temperatur und/oder der pH-Wert und/oder der Durchfluss (d.h. die Durchflussmenge des Wassers) und/oder die Trübung gemessen werden. Hierdurch kann die Bestimmung der Konzentration von Chlorat/Desinfektionswirkstoffen mit größerer Genauigkeit erfolgen, da der Einfluss dieser Größen auf den in Schritt (b) gewonnenen amperometrischen Messwert korrigiert werden kann.

In einer Ausführungsform können die Messwerte des 2- oder 3-Elektrodensystem und der optionalen Messung von weiteren Parametern, wie Konzentration von Desinfektionswirkstoffen, Temperatur und/oder pH-Wert und/oder Durchfluss und/oder Trübung in einer Steuer- oder Regeleinheit verarbeitet werden. Eine solche Verarbeitung kann die Visualisierung der Messwerte umfassen, beispielsweise mit einem Display oder einer LED-Zeile mit Ampelfarben. In einigen Ausführungsformen der Erfindung können die Messwerte der Messelektrode zur Regelung der Zugabe von Desinfektionsmittel verwendet werden.

In einer Ausführungsform können die Messwerte der 2- oder 3-Elektrodensystem und optional auch die Messwerte der Messung weiterer Parameter, wie Konzentration von Desinfektionswirkstoffen, Temperatur und/oder pH-Wert und/oder Durchfluss und/oder Trübung in einem Datenspeicher gespeichert werden. Der Datenspeicher kann dazu mit der Steuer- oder Regeleinheit verbunden sein.

In einigen Ausführungsformen der Erfindung können die Messwerte des 2- oder 3-Elektrodensystems und optional auch die Messwerte der Messung der Konzentration von Desinfektionswirkstoffen, Temperatur und/oder pH-Wert und/oder Durchfluss mit einer Datenfernübertragungseinrichtung übertragen werden Hierzu kann die Steuer- oder Regeleinrichtung mit einer Kabelverbindung oder einer drahtlosen Verbindung mit einer räumlich abgesetzten Empfangseinheit verbunden sein.

Mit vorstehenden Bauelementen ist eine Steuerung oder eine Regelung oder/und eine Überwachung des erfindungsgemäßen Verfahrens möglich. So kann beispielsweise ein Sollwert oder ein Sollwertbereich mit Ober- und Untergrenzen festgelegt werden. Wird dieser über- oder unterschritten, kann ein Signal erzeugt werden, so dass dann beispielsweise die Desinfektionsmittelmenge geregelt werden kann. Auf diese Weise können Chlorat-Grenzwerte bestimmt und überwacht werden.

Gegenstand der vorliegenden Erfindung ist ferner eine Vorrichtung, mit der das vorstehend beschriebene Verfahren durchgeführt werden kann. Das vorstehend beschriebene Verfahren kann dazu herangezogen werden, die Bau- und Funktionsweise der nachfolgend beschriebenen erfindungsgemäßen Vorrichtung näher zu erläutern.

Die erfindungsgemäße Vorrichtung ist eine Vorrichtung zum Kalibrieren eines 2- oder 3-Elektrodensystems mit den Merkmalen des Anspruchs 2.

In einer Ausführungsform weist die Vorrichtung weiterhin zumindest einen Temperaturfühler und/oder zumindest eine pH-Messelektrode und/oder zumindest einen Durchflussmesser und/oder zumindest einen Trübungsmesser und/oder eine Messsonde für Chlor, Chlorit, Hypochlorit oder Chlordioxid und/oder eine Messsonde für die Leitfähigkeitsmessung und/oder eine RedOx-Messsonde und/oder einer Messsonde für ein Biozid auf.

In einer Ausführungsform enthält die Einrichtung zum Abzweigen von zu untersuchendem Wasser zumindest einen Regler oder zumindest ein Ventil, die manuell, elektrisch oder pneumatisch bedient werden können, beispielsweise ein Dreiwegeventil.

In einer weiteren Ausführungsform weist die Vorrichtung eine Steuer- oder Regeleinrichtung auf, in der die Messwerte des 2- oder 3-Elektrodensystems sowie gegebenenfalls des Temperaturfühlers, der pH-Messelektrode, des Trübungsmessers und/oder des Durchflussmessers und/oder eine Messsonde für Chlor, Chlorit, Hypochlorit oder Chlordioxid und/oder eine Messsonde für die Leitfähigkeitsmessung und/oder eine RedOx-Messsonde und/oder eine Messsonde für ein Biozid verarbeitet werden.

Ferner kann in einer Ausführungsform die Vorrichtung einen Datenspeicher aufweisen, in welchem die Messwerte des 2- oder 3-Elektrodensystems sowie gegebenenfalls des Temperaturfühlers, der pH-Messelektrode, des Trübungsmessers und/oder des Durchflussmessers und/oder der Messsonde für Chlor, Chlorit, Hypochlorit oder Chlordioxid und/oder der Messsonde für die Leitfähigkeitsmessung und/oder der RedOx-Messsonde und/oder der Messsonde für ein Biozid gespeichert werden. Weiterhin kann die Vorrichtung eine Datenfernübertragungseinrichtung aufweisen.

In einer Ausführungsform kann die Vorrichtung weiterhin einen Vorratsbehälter aufweisen. Dieser Vorratsbehälter kann sich im Kreislauf befinden, in dem das abgezweigte Wasser transportiert wird. Dazu kann an einer Stelle in den Vorratsbehälter eine Zufuhrleitung hinein und an einer anderen Stelle eine Entnahmeleitung aus dem Behälter herausführen. Die Entnahmeleitung kann mit der Saugseite einer elektrischen Pumpe gekoppelt sein, welche das entnommene Wasser im zweiten Betriebszustand, welcher den Kalibrierbetrieb darstellt, in den Kreislauf mit dem 2- oder 3-Elektrodensystem einspeist und über die Zufuhrleitung in den Vorratsbehälter zurückführt. Hierdurch wird eine blasenfreie und konstante Strömung im Kreislauf ermöglicht.

Weiterhin kann die Entnahmevorrichtung mit dem Vorratsbehälter verbunden sein, so dass sichergestellt ist, dass das zur Bestimmung des Normals beispielsweise mit der DPD-Methode entnommene Wasser demselben Kreislauf entstammt und dieselbe Konzentration von Desinfektionswirkstoffen enthält.

Nachfolgend soll die Erfindung anhand von Figuren ohne Beschränkung des allgemeinen Erfindungsgedankens näher erläutert werden. Dabei zeigt
Fig. 1 eine Vorrichtung zur amperometrischen Messung der Konzentration von Chlorat in Wasser im ersten Betriebszustand, welcher den Normalbetrieb darstellt.
Fig. 2 zeigt die Vorrichtung gemäß Figur 1 im zweiten Betriebszustand, welcher den Kalibrierbetrieb darstellt.
Figur 3 zeigt eine Messvorrichtung mit einer darin integrierten Pumpe.

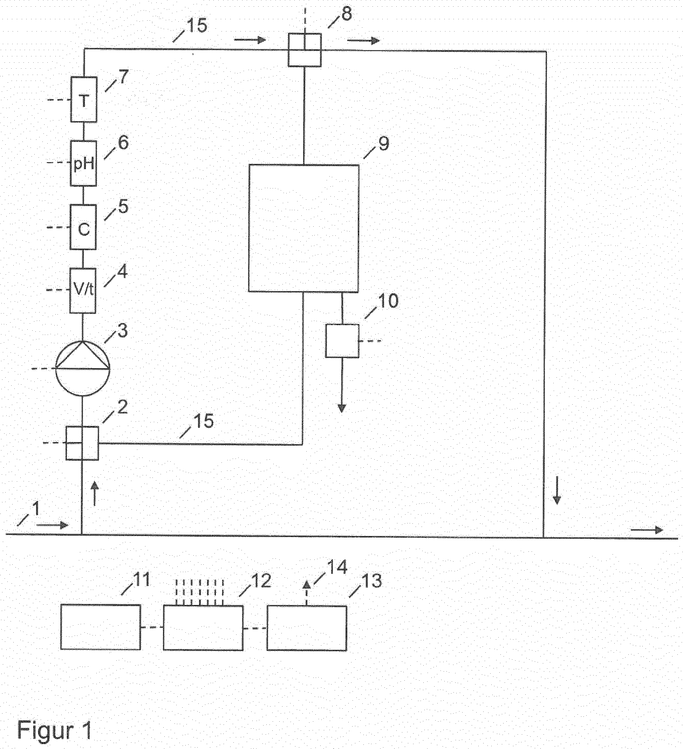

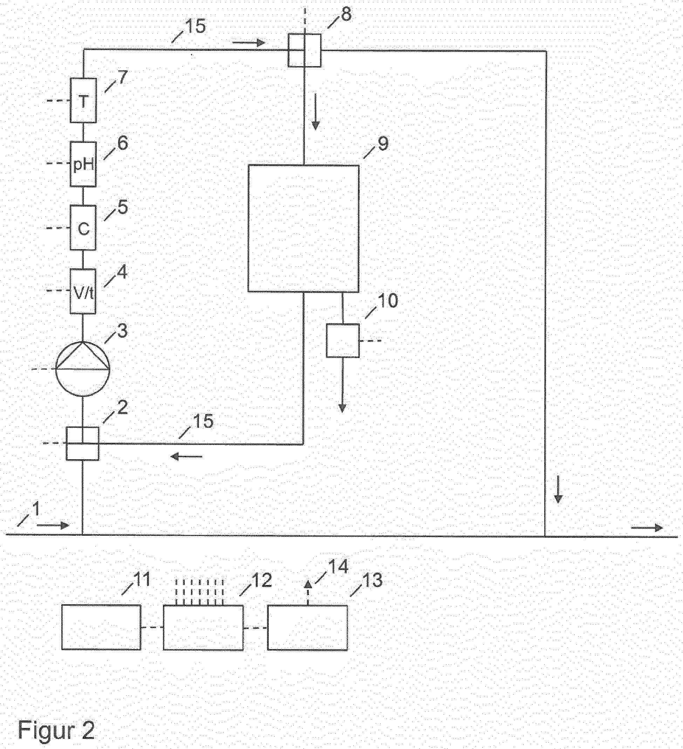

Anhand der Figuren 1 und 2 wird eine erfindungsgemäße Vorrichtung zum Kalibrieren und das Verfahren zu deren Betrieb näher erläutert. Dabei zeigt Figur 1 den ersten Betriebszustand, welcher den Normalbetrieb darstellt und Figur 2 den zweiten Betriebszustand, welcher den Kalibrierbetrieb darstellt. Daraus ergeben sich auch weiterführende technische Details zur Bestimmung von Chlorat.

Figur 1 zeigt eine Wasserleitung 1, über welche Wasser aus einer nicht dargestellte Wasserversorgungseinrichtung, beispielsweise einem Hochbehälter oder einer Quellfassung, einem nicht dargestellten Verbraucher zugeführt werden kann, beispielsweise einem Haushalt oder einer Gemeinde. In anderen Ausführungsformen der Erfindung kann Wasser einem Schwimmbecken über die Leitung 1 entnommen werden und nach Durchlauf durch eine Filteranlage dem Schwimmbecken wieder zugeführt werden.

In der Wasserleitung 1 befindet sich eine Einrichtung 2 zum Abzweigen eines Teilstromes des zu untersuchenden Wassers. In einigen Ausführungsformen der Erfindung kann die Einrichtung 2 ein Dreiwegeventil enthalten oder daraus bestehen. Die Einrichtung 2 kann manuell oder elektrisch oder pneumatisch bedient werden. Die Einrichtung 2 kann an eine elektronische Steuer- oder Regeleinrichtung 12 angeschlossen sein, um einen vollautomatischen Betrieb des erfindungsgemäßen Verfahrens zu ermöglichen. Die Einrichtung 2 zum Abzweigen eines Teilstromes ist mit der Wasserleitung 1 über ein T-Stück verbunden.

Im dargestellten Ausführungsbeispiel strömt das abgezweigte Wasser hinter der Einrichtung 2 durch eine Leitung, in welcher zumindest ein 2- oder 3-Elektrodensystem 5 angeordnet ist, mit welcher die Konzentration von Chlorat bestimmt wird. Optional können weitere Komponenten in dieser Leitung angeordnet sein, beispielsweise zumindest eine Pumpe 3 und/oder zumindest ein Durchflussmesser 4 und/oder zumindest eine pH-Messelektrode 6 und/oder zumindest ein Temperaturfühler 7 und/oder zumindest ein zweites Dreiwegeventil 8, das manuell, elektrisch oder pneumatisch bedient werden kann, und/oder zumindest ein Vorratsbehälter 9.

Der Vorratsbehälter 9 kann mit einer optionalen Entnahmevorrichtung 10 ausgestattet sein. Im dargestellten Ausführungsbeispiel ist die Entnahmevorrichtung 10 als Ablass- und Probeentnahmeventil ausgebildet. Der Vorratsbehälter 9 weist einen Einlauf auf, welcher dazu eingerichtet ist, den Vorratsbehälter 9 über einen Ausgang des zweiten Dreiwegeventils 8 zu befüllen. Weiterhin weist der Vorratsbehälter 9 einen Auslauf auf. Der Auslauf ist über eine Leitung 15 mit einem weiteren Eingang der Einrichtung 2 zum Abzweigen eines Teilstromes verbunden.

Optional kann die Vorrichtung eine Steuer- oder Regeleinrichtung 12 enthalten. Die Einrichtung 2, das 2- oder 3-Elektrodensystem 5, die Pumpe 3, der Durchflussmesser 4, die pH-Messelektrode 6, der Temperaturfühler 7 und/oder das zweite Dreiwegeventil 8 können über Strom- und/oder Datenleitungen mit der Steuer- oder Regeleinrichtung 12 verbunden sein. Die elektrischen Leitungen sind in den Figuren als gestrichelte Linien dargestellt, wobei aber deren Verbindung aus Gründen der Übersichtlichkeit nicht gezeigt ist. Alternativ kann die Verbindung der Messeinrichtungen 4, 5, 6 und 7 mit der Steuer- oder Regeleinrichtung 12 auch über eine Funkverbindung erfolgen.

Die Steuer- oder Regeleinrichtung 12 kann mit einem optionalen Datenspeicher 11 und einer optionalen Datenfernübertragungseinrichtung 13 verbunden sein. In anderen Ausführungsformen der Erfindung kann die Steuer- oder Regeleinrichtung 12 einen optionalen Datenspeicher 11 und/oder eine optionale Datenfernübertragungseinrichtung 13 enthalten.

Die Datenfernübertragungseinrichtung 13 kann eine optionale Antenne 14 zur drahtlosen Datenübertragung enthalten oder Daten über ein Telefon- oder Computernetzwerk mittels einer optischen oder elektrischen Kabelverbindung übertragen. In einigen Ausführungsformen kann die erfindungsgemäße Vorrichtung auch ohne Datenfernübertragung autark betrieben werden. Vor Ort können Messwerte angezeigt, ausgelesen und Steuerbefehle eingegeben werden. Alternativ dazu kann die erfindungsgemäße Vorrichtung über die Datenfernübertragungseinrichtung 13 gesteuert, ausgelesen und in ein räumlich vernetztes Steuerungs- und Überwachungssystem eingebunden werden.

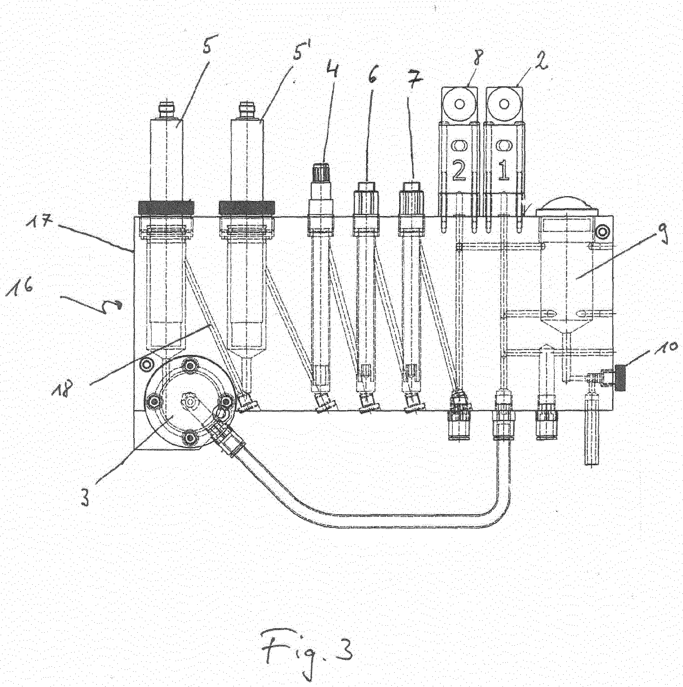

Alle Bauteile können in einem abschließbaren und manipulationssicheren Gehäuse eingebaut sein, welches nachfolgend in Fig. 3 gezeigt ist.

In dem in Figur 1 gezeigten ersten Betriebszustand wird zumindest ein Teilstrom des in der Wasserleitung 1 ankommenden Wassers über das T-Stück und die Einrichtung 2 in die Leitung mit dem 2- oder 3-Elektrodensystem 5 geführt. In einigen Ausführungsformen der Erfindung kann dieser Teilstrom auch die gesamte in der Wasserleitung 1 ankommende Wassermenge umfassen. Der im ersten Betriebszustand vorliegende Wasserfluss ist in Figur 1 mit Pfeilen dargestellt.

Somit wird im Normalbetrieb das abgezweigte Wasser kontinuierlich entnommen und über das 2- oder 3-Elektrodensystem 5 und die weiteren, optionalen Messeinrichtungen 4, 6 und 7 geleitet. Das 2- oder 3-Elektrodensystem 5 und die weiteren Messeinrichtungen 4, 6, 7 sind in Fig. 1 seriell geschaltet. Sie können alternativ dazu auch parallel geschaltet sein. Sodann wird das abgezweigte Wasser über das zweite Dreiwegeventil 8 einer Rücklaufleitung zugeführt, welche stromabwärts des zur Entnahme verwendeten T-Stücks in der Wasserleitung 1 mündet. Der Vorratsbehälter 9 ist nicht in Betrieb und kann vorzugsweise entleert sein. Die Pumpe 3 kann zur Unterstützung des Wasserstromes über die Messeinrichtungen 4, 5, 6 und 7 verwendet werden oder aber auch bei ausreichendem Wasserdruck über eine nicht dargestellte Bypassleitung umgangen werden oder passiv durchströmt werden.

Die Messsignale des Durchflussmessers 4, des 2- oder 3-Elektrodensystems 5, der pH-Messelektrode 6 und des Temperaturfühlers 7 werden zur zentralen Steuer- oder Regeleinrichtung 12 geleitet, verarbeitet, ausgewertet, angezeigt und/oder im Datenspeicher 11 gespeichert. Über die Datenfernübertragungseinrichtung 13 mit Antenne 14 können sie auch an entfernten Orten angezeigt und bearbeitet werden. So ist eine zeitlich lückenlose Überwachung der Konzentration der Desinfektionswirkstoffe im Wasser möglich.

Bei Überschreitungen eines vorgebbaren Grenzwertes kann ein Alarm ausgelöst werden, um einen Bediener auf die Fehlfunktion hinzuweisen, beispielsweise dass die Chlorat-Konzentration einen vorgegebenen Grenzwert, der sich z.B. aus entsprechenden gesetzlichen Vorschriften ergeben kann, überschritten wird. Alternativ oder zusätzlich kann eine nicht dargestellte Dosiereinrichtung des Desinfektionsmittels beeinflusst werden, um die Zugabe reduzieren oder einzustellen. Bei Unterschreitungen eines vorgebbaren Grenzwertes kann ebenfalls ein Alarm ausgelöst werden, um einen Bediener auf die Fehlfunktion der Desinfektion hinzuweisen. Alternativ oder zusätzlich kann eine nicht dargestellte Dosiereinrichtung für das Desinfektionsmittel beeinflusst werden und die Zugabe erhöht oder vermindert werden. Durch die kontinuierliche Überwachung kann einer Unterversorgung mit mangelhafter Desinfektionsleistung ebenso wie einer Überversorgung mit Schädigung der Wasserverbraucher wirksam vorgebeugt werden. Letzteres ist vor allem bei der Einhaltung von Chlorat-Grenzwerten zu beachten.

Um das 2- oder 3-Elektrodensystem 5 erstmalig oder regelmäßig im Betrieb zu kalibrieren, kann die Vorrichtung in den zweiten Betriebszustand geschaltet werden. Dieser ist in Figur 2 dargestellt. Gleiche Bestandteile der Erfindung sind mit gleichen Bezugszeichen versehen, so dass sich die nachfolgende Beschreibung auf die wesentlichen Unterschiede beschränkt. Auch in Figur 2 ist der Wasserfluss durch Pfeile symbolisiert.

Um in den zweiten Betriebszustand zu schalten, schaltet zunächst das zweite Dreiwegeventil 8, sodass die Rücklaufleitung abgetrennt und der Vorratsbehälter 9 gefüllt wird. Bei Erreichen eines vorgebbaren Füllstandes wird die Einrichtung 2 so geschaltet, dass der Ausgang des Vorratsbehälters 9 über die Leitung 15 mit dem Eingang der Pumpe 3 verbunden wird. Somit wird ein geschlossener Kreislauf abgezweigten Wassers etabliert, nämlich vom Vorratsbehälter 9 über die Pumpe 3, den Durchflussmesser 4, dem 2- oder 3-Elektrodensystem 5, der pH-Messelektrode 5 und dem Temperaturfühler 7 und das zweite Dreiwegeventil 8 zurück in den Vorratsbehälter 9. Das von der Wasserversorgungseinrichtung, beispielsweise einem Hochbehälter oder einer Quellfassung, an die Verbraucher gelieferte Wasser strömt im zweiten Betriebszustand vollständig in der Wasserleitung 1 an der erfindungsgemäßen Vorrichtung vorbei.

Durch den geschlossenen Kreislauf abgezweigten Wassers ist sichergestellt, dass sich die Konzentration der Desinfektionswirkstoffe im abgezweigten Wasser nicht ändert. Wenn zumindest der Messwert des 2- oder 3-Elektrodensystems 5, vorzugsweise aber auch die Messwerte des Durchflussmessers 4, der pH-Messelektrode 6 und des Temperaturfühlers 7, konstant sind, wird die Entnahmevorrichtung 10 des Vorratsbehälters 9 geöffnet, sodass eine Probe entnommen werden kann. In dieser Probe wird die Konzentration von Chlorat mit einem alternativen, als Normal dienenden Verfahren bestimmt. Beispielsweise kann dies photometrisch mit der DPD-Methode erfolgen. Der so bestimmte Wert wird sodann zur Kalibrierung des 2- oder 3-Elektrodensystems verwendet.

Während der Bestimmung des Normals zirkuliert das abgezweigte Wasser weiter über das 2- oder 3-Elektrodensystem 5, den Durchflussmesser 4, die pH-Messelektrode 6 und den Temperaturfühler 7. An dem 2- oder 3-Elektrodensystem liegen dadurch konstante Werte für Strömung, Konzentration von Chlorat, pH-Wert und Temperatur an. Die Kalibrierung erfolgt so zuverlässig.

Danach wird das zweite Dreiwegeventil 8 umgeschaltet, sodass das Wasser über die Rücklaufleitung in die Wasserleitung 1 abfließen kann. Kurz darauf kann auch die Einrichtung 2 wieder umgeschaltet werden, um Wasser aus der Wasserleitung 1 zu entnehmen. Der Vorratsbehälter 9 kann entweder über die Einrichtung 2 und das Dreiwegeventil 8 in die Rücklaufleitung entleert werden oder aber über Entnahmevorrichtung 10. Das Wasser aus der Leitung 1 kann sofort weiterhin kontinuierlich überwacht werden. In einigen Ausführungsformen der Erfindung kann der vorstehend beschriebene Ablauf mehrfach wiederholt werden, um auf diese Weise eine Mehrzahl von Kalibrierpunkten bei unterschiedlichen Messwerten der Konzentration der Desinfektionswirkstoffe zu erhalten.

In der Fig. 3 ist eine Ausführungsformen der Messvorrichtung 16 gezeigt. Gleiche Bauteile sind mit gleichen Bezugszeichen versehen, so dass auf vorstehende Ausführungen verwiesen wird. Die Messvorrichtung 16 weist ein Gehäuse 17 auf, in dem die Komponenten der Messvorrichtung 16 zumindest teilweise eingebaut sein können. Das Gehäuse 17 kann aus Metall, Kunststoff oder eine Kombination dieser beiden bestehen oder diese umfassen. Zum Transport des zu untersuchenden Wassers in der vorstehend beschriebenen Weise ist eine Pumpe 3 vorgesehen, die in der in Fig. 3 gezeigten Ausführungsform in das Gehäuse integriert ist. Alternativ kann die Pumpe sich aber auch außerhalb des Gehäuses befinden. Des weiteren weist die Messvorrichtung 16 das 2- oder 3-Elektrodensystem zur Bestimmung von Chlorat 5 auf. Ferner ist optional eine weitere Messsonde 5' vorgesehen, mit der Chlor, Chlorit, Chlordioxid, Ozon oder Peressigsäure bestimmt werden können. Die Messvorrichtung 16 kann auch optional mehrere dieser weiteren Messsonden 5' aufweisen. Ferner können optional die weiteren Messeinrichtungen 4, 6 und 7 vorliegen. Die Durchflusswege 18 können schräg verlaufen. Sie können aber auch unten, terminal bzw. subterminal und oben an den Bohrungsenden der Aufnahmebereiche horizontal verlaufen. Mit schräg verlaufenden Durchflusswegen können Stagnationen vermieden werden.

Selbstverständlich ist die Erfindung nicht auf die in den Figuren dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Beschreibung oder die Ansprüche ,erste' und ,zweite' Merkmale definieren, so dient dies der Unterscheidung gleichartiger Merkmale, ohne eine Rangfolge festzulegen.

## Patentansprüche

1. Verfahren zum Kalibrieren eines 2- oder 3-Elektrodensystems (5), welches zur Bestimmung der Konzentration von Chlorat in Wasser eingesetzt wird, **dadurch gekennzeichnet, dass**
(a) von einer Wasserleitung (1) zu untersuchendendes Wasser abgezweigt und an dem 2- oder 3-Elektrodensystem (5) vorbeigeleitet wird, wobei das 2-Elektrodensystem (5) eine Messelektrode, die aus Gold besteht oder dieses umfasst, und eine gemeinsame Gegen- und Bezugselektrode aufweist, die aus Silber besteht oder dieses umfasst und mit einem Überzug, der Silberhalogenid umfasst oder daraus besteht, versehen ist, wobei zwischen den beiden Elektroden eine Vorspannung angelegt ist, oder
wobei das 3-Elektrodensystem (5) eine Messelektrode, die aus Gold besteht oder dieses umfasst, eine Bezugselektrode, die aus Silber besteht oder dieses umfasst und mit einem Überzug versehen ist, der aus Silberhalogenid besteht oder dieses umfasst, sowie eine Gegenelektrode aufweist, die aus Edelstahl besteht oder diesen umfasst, wobei mittels eines Potentiostaten die Messelektrode auf einem Arbeitspotential gehalten wird und wobei dieses Vorbeileiten in einem Kreislauf erfolgt,
(b) ein Messwert des 2- oder 3-Elektrodensystems (5) bestimmt wird,
(c) die Konzentration von Chlorat des abgezweigten Wassers mit einem weiteren Verfahren ermittelt wird, und
(d) die in Schritt (c) ermittelte Konzentration von Chlorat als Normal für die Kalibrierung des 2- oder 3-Elektrodensystems (5) verwendet wird.

2. Vorrichtung zum Kalibrieren eines 2- oder 3-Elektrodensystems zur Bestimmung von Chlorat (5)zur Verwendung in dem Verfahren nach Anspruch 1, **dadurch gekennzeichnet, dass** sie umfasst:
(i) eine Einrichtung zum Abzweigen (2) von zu untersuchendem Wasser aus einer Wasserleitung (1)
(ii) ein 2- oder 3-Elektrodensystem (5), wobei das 2-Elektrodensystem (5) eine Messelektrode, die aus Gold besteht oder dieses umfasst, und eine gemeinsame Gegen- und Bezugselektrode aufweist, die aus Silber besteht oder dieses umfasst und mit einem Überzug, der Silberhalogenid umfasst oder daraus besteht, versehen ist, wobei zwischen den beiden Elektroden eine Vorspannung angelegt ist, oder
wobei das 3-Elektrodensystem (5) eine Messelektrode, die aus Gold besteht oder dieses umfasst, eine Bezugselektrode, die aus Silber besteht oder dieses umfasst und mit einem Überzug versehen ist, der aus Silberhalogenid besteht oder dieses umfasst, sowie eine Gegenelektrode aufweist, die aus Edelstahl besteht oder diesen umfasst, wobei mittels eines Potentiostaten die Messelektrode auf einem Arbeitspotential gehalten wird,
(iii) eine Leitung (15), in der das abgezweigte Wasser im Kreislauf an dem 2- oder 3-Elektrodensystem (5) vorbeigeführt wird, und
(iv) eine Entnahmevorrichtung (10), um einen Teil des abgezweigten Wassers zu entnehmen.

## Claims

1. Method for calibrating a 2- or 3-electrode system (5) which is used to determine the concentration of chlorate in water, **characterized in that**
(a) water from a water line (1), which shall be analyzed, is branched off and guided past the 2- or 3-electrode system (5), the 2-electrode system (5) including a measuring electrode which consists of or comprises gold, and a joint counter and reference electrode which consists of or comprises silver and is provided with a coating which comprises or consists of silver halide, a bias voltage being applied between the two electrodes, or
the 3-electrode system (5) including a measuring electrode, which consists of or comprises gold, a reference electrode which consists of or comprises silver and is provided with a coating which consists of or comprises silver halide, as well as has a counter electrode which consists of or comprises stainless steel, the measuring electrode being maintained at a working potential by means of a potentiostat and this guidance being carried out **in** a circuit,
(b) a measuring value of the 2- or 3-electrode system (5) is determined,
(c) the concentration of chlorate **in** the branched-off water is determined by means of a further method, and
(d) the concentration of chlorate determined in step (c) is used as a standard for calibrating the 2- or 3-electrode system (5).

2. Apparatus for calibrating a 2- or 3-electrode system for determining chlorate (5) for use **in** the method according to claim 1, **characterized in that** it comprises:
(i) a device (2) for branching off water to be analyzed from a water line (1)
(ii) a 2- or 3-electrode system (5), the 2-electrode system (5) including a measuring electrode which consists of or comprises gold and a joint counter and reference electrode which consists of or comprises silver and is provided with a coating which comprises or consists of silver halide, a bias voltage being applied between the two electrodes, or
the 3-electrode system (5) including a measuring electrode which consists of or comprises gold, a reference electrode which consists of or comprises silver and is provided with a coating which consists of or comprises silver halide, as well as a counter electrode which consists of or comprises stainless steel, the measuring electrode being maintained at a working potential by means of a potentiostat,
(iii) a line (15) within which the branched-off water is guided in a circuit past the 2- or 3-electrode system (5), and
(iv) a removal device (10) for removing a portion of the branched-off water.

## Revendications

1. Procédé d'étalonnage d'un système à 2 ou 3 électrodes (5), utilisé pour déterminer la concentration en chlorate dans l'eau,
**caractérisé en ce que**
(a) l'eau à analyser est dérivée à partir d'une conduite d'eau (1) et est acheminée pour passer devant le système à 2 ou 3 électrodes (5), le système à 2 électrodes (5) comportant une électrode de mesure, constituée d'or ou en contenant, et une électrode commune servant à la fois de contre-électrode et d'électrode de référence, constituée d'argent ou en contenant et pourvue d'un revêtement contenant de l'halogénure d'argent ou constitué de celui-ci,
une tension de polarisation étant appliquée entre les deux électrodes, ou le système à 3 électrodes (5) comportant une électrode de mesure, constituée d'or ou en contenant, une électrode de référence constituée d'argent ou en contenant et pourvue d'un revêtement constitué d'halogénure d'argent ou en contenant, ainsi qu'une contre-électrode constituée d'acier inoxydable ou en contenant,
l'électrode de mesure étant maintenue à un potentiel de travail à l'aide d'un potentiostat et l'acheminement s'effectuant dans un circuit,
(b) une valeur de mesure du système à 2 ou 3 électrodes (5) est déterminée,
(c) la concentration en chlorate de l'eau dérivée est déterminée à l'aide d'un autre procédé, et
(d) la concentration en chlorate déterminée à l'étape (c) est utilisée comme étalon pour l'étalonnage du système à 2 ou 3 électrodes (5).

2. Dispositif destiné à l'étalonnage d'un système à 2 ou 3 électrodes pour la détermination du chlorate (5), destiné à être utilisé dans le procédé selon la revendication 1,
**caractérisé en ce qu'**il comprend :
(i) un dispositif de dérivation (2) de l'eau à analyser à partir d'une conduite d'eau (1),
(ii) un système à 2 ou 3 électrodes (5), le système à 2 électrodes (5) comportant une électrode de mesure constituée d'or ou en contenant, et une électrode commune servant à la fois de contre-électrode et d'électrode de référence, constituée d'argent ou en contenant et pourvue d'un revêtement contenant de l'halogénure d'argent ou constitué de celui-ci,
une tension de polarisation étant appliquée entre les deux électrodes, ou
le système à 3 électrodes (5) comportant une électrode de mesure constituée d'or ou en contenant, une électrode de référence constituée d'argent ou en contenant et pourvue d'un revêtement constitué d'halogénure d'argent ou en contenant, ainsi qu'une contre-électrode constituée d'acier inoxydable ou en contenant,
l'électrode de mesure étant maintenue à un potentiel de travail à l'aide d'un potentiostat,
(iii) une conduite (15) dans laquelle l'eau dérivée est acheminée dans un circuit pour passer devant le système à 2 ou 3 électrodes (5), et
(iv) un dispositif de prélèvement (10) destiné à prélever une partie de l'eau dérivée.
